(19) **Europäisches Patentamt**
**European Patent Office**
**Office européen des brevets**

(11) **EP 4 737 446 A1**

(12) **EUROPEAN PATENT APPLICATION**

(43) Date of publication:
**06.05.2026 Bulletin 2026/19**

(21) Application number: **24383185.6**

(22) Date of filing: **30.10.2024**

(51) International Patent Classification (IPC):
**C07D 307/33** $^{(2006.01)}$   **C07C 67/08** $^{(2006.01)}$

(52) Cooperative Patent Classification (CPC):
**C07D 307/33**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicants:
• **Consejo Superior de Investigaciones Científicas (CSIC)**
**28006 Madrid (ES)**
• **Universitat de Barcelona**
**08028 Barcelona (ES)**
• **Fundació Institut Català de Nanociència i Nanotecnologia (ICN2)**
**08193 Bellaterra (ES)**
• **Institució Catalana de Recerca i Estudis Avançats**
**(ICREA)**
**08010 Barcelona (ES)**

(72) Inventors:
• **Fons Cervera, Arnau**
**Barcelona (ES)**
• **Sepúlveda Martínez, Borja**
**Barcelona (ES)**
• **Serrà Ramos, Albert**
**Barcelona (ES)**
• **Gómez Valentín, Elvira**
**Barcelona (ES)**
• **Nogues Sanmiquel, Josep**
**Barcelona (ES)**
• **Bujaldón Carbó, Roger**
**Barcelona (ES)**

(74) Representative: **Pons IP**
**Glorieta Rubén Darío 4**
**28010 Madrid (ES)**

(54) **PROCEDURE FOR THE CONVERSION OF GAMMA-KETOACIDS TO GAMMA-LACTONES VIA PHOTOTHERMAL CATALYSIS**

(57)    The present invention relates to a procedure for the conversion of γ-ketoacids , such as levulinic acid, to γ-lactones in the presence of a Ni-based catalyst and isopropanol as hydrogen source and solvent via photothermal process using UV to NIR light as energy source (such as solar and laser). This is a sustainable, economically and environmentally viable procedure that has demonstrated nearly complete conversion in less than 2h under moderate conditions.

EP 4 737 446 A1

## Description

The invention relates to a sustainable procedure to obtain γ-lactones, more particularly, γ-valerolactone, which, apart from being a platform to other high-value chemicals, it has applicability itself as a biofuel, green solvent or even in perfumery. γ-lactones are synthesized through a hydrogenation reaction from γ-ketoacids via photothermal catalysis.

## BACKGROUND ART

Reducing the overreliance on fossil fuels and curbing the steady rise of $CO_2$ emissions has grown into a pressing global priority to address the climate crisis. In this pursuit, biomass valorization emerges as an engaging approach for transitioning away from fossil fuels towards a more sustainable economy. Biomass originates as a residue from activities as diverse as agriculture, livestock-related industries, and the management of forested areas, which regards it as the only renewable raw material that can be readily converted into organic molecules. Lignocellulosic wastes indeed hold significant interest in their derivatization to biofuels and related value-added compounds. Recognizing this perspective, forest management must transcend ecological considerations alone and adopt a holistic approach. Forests can serve not solely as ecosystems but also as clean and sustainable factories. Consequently, the development of novel strategies that pave the way towards a biomass-based system, in which bioeconomy and circularity represent the main principles, stands as a prime objective.

The composition of lignocellulosic biomass majorly comprises cellulose (35-50%), hemicellulose (20-40%) and lignin (10-25%). The formers can be transformed into synthetic building blocks of interest, such as 5-hydroxymethylfurfural (5-HMF) and levulinic acid (LA), through successive physicochemical processes that have been extensively reported.

γ-Valerolactone (GVL) is one of the most promising biomass derivatives, considering its applications as biofuel, alternative greener solvent, precursor of biopolymers, as well as its value in perfumery. Accessing an economically viable process for a sustainable production from biomass has inevitably become a pivotal area of research. The synthesis of γ-valerolactone implies the hydrogenation of the ketone group of levulinic acid, which is generally conducted with molecular hydrogen, and its subsequent intramolecular esterification. The main production of hydrogen, however, still relies on non-renewable sources, which critically hampers the sustainability expected from this procedure. This method also involves working under high pressure to promote the reaction, which complicates the requirements of the facilities and industrial plants. A well-known strategy to circumvent these factors is the incorporation of alternative biomass-derived reducing agents in which hydrogen is supplied/generated in situ. Two prime examples are formic acid, which decomposes catalytically into hydrogen and carbon dioxide, and alcohols, particularly secondary ones, which undergo a catalytic hydrogen transfer via the Meerwein-Ponndorf-Verley reaction. The former is especially attractive since it is produced equimolarly along with levulinic acid during the acid-mediated rehydration and subsequent oxidation of 5-HMF. Thus, it would be continuously supplied into the reaction medium in a one-pot synthetic strategy starting from cellulose, apart from being a biomass-derived reagent.

Another key challenge to be considered for this process is the high energy consumption associated with elevated working temperatures to carry out the hydrogenation reaction. The nature and characteristics of the catalyst are particularly decisive at this point. A vast range of catalytic platforms based on diverse noble and non-noble metals have been reported so far [1) Valentini, F.; Marrocchi, A.; Vaccaro, L. Adv. Energy Mater. 2022, 12, 2103362. 2) Hijazi, A.; Khalaf, N.; Kwapinski, W.; Leahy, J. J. RSC Adv. 2022, 12, 13673-13694.]. The scope of temperatures needed is equally varied, but generally goes up to 180 °C, which makes it highly energy demanding. Therefore, developing new technologies to reduce energy consumption is a major challenge. In this context, photothermocatalytic reactions could provide an interesting alternative to drastically reduce the necessary input energy. Photothermocatalysis is based on the use of an intense light source to produce the heat necessary for the catalyzed reaction to progress. Photothermal catalysis can follow two different catalytic pathways: thermochemical or photochemical. In the thermochemical pathway, the photothermal catalyst interacts with the incident light and then converts the photon energy into thermal energy by exciting vibrational states. Noble metal plasmonic nanoparticles and carbonaceous materials are often used as photothermal catalysts [1) Fang, S.; Hu, Y. H. Chem. Soc. Rev. 2022, 51, 3609-3647. 2) Zhang, J.; Chen, H.; Duan, X.; Sun, H.; Wang, S. Mater. Today 2023, 68, 234-253.]. Noble metals nanostructures can generate localized surface plasmon resonances, allowing for the efficient conversion of light into heat. However, in addition to their high cost, the shape, morphology, size and composition of the metal nanoparticles greatly determine their narrow spectral absorption band, thereby limiting the heating efficiency in broadband light sources, such as sunlight. On the other hand, various forms of nanocarbon materials such as graphene, graphene oxide (GO), carbon nanotubes (CNT) or reduced graphene oxide (rGO) show broadband light absorption, high stability and good thermal conductivity, although they suffer from high cost and limited activity in certain hydrogenation reactions. Therefore, it is necessary to find new cost-effective, abundant and highly stable photothermocatalytic processes compatible with sunlight to drastically reduce the economic and environmental cost of hydrogenation reactions.

With this aim, the present invention proposes a sustainable, economically and environmentally viable solution for



**EP 4 737 446 A1**

the hydrogenation of γ-ketoacids , such as levulinic acid, to produce the corresponding γ-lactones, such as γ-valerolactone, being compatible with sunlight activation.

**SUMMARY OF THE INVENTION**

In this invention, it is presented a photothermal procedure based on Ni-based catalysts as an alternative energy efficient pathway for the generation of hydrogenation reactions. In particular, the highly efficient conversion of γ-ketoacids, preferably levulinic acid, a waste product of lignocellulose biomass, into γ-lactones, preferably γ-valerolactone, has been demonstrated.

γ-ketoacids (also called Gamma-keto acids, Gamma-ketoacids, or 4-oxoacids) are organic compounds that contain a carboxylic acid group (-COOH) and a ketone group (>C=O) and have the ketone group at the third carbon from the carboxylic acid.

The production of γ-lactones is based on a dual strategy: i) reducing the energy demand of the process by utilizing light source to induce the thermocatalytic process, and (heating is achieved by applying light) ii) the use Ni-based catalyst, such as Raney® Ni (also referred simply as Raney Ni), a highly accessible and inexpensive catalyst widely recognized in hydrogenation processes by exploiting its highly damped plasmonic behavior to generate highly efficient local photothermal heating, even in micron size particles, and graphite microstructures functionalized with Ni-P by electroless deposition, referred as graphite/Ni-P.

Then, a first aspect of the present invention relates to a procedure for the conversion of a γ-ketoacid to the corresponding γ-lactone via photothermal catalysis comprising contacting the γ-ketoacid with a Ni-based catalyst and with isopropanol, which is used as a hydrogen source and solvent, and photothermally heating at a temperature between 100 and 150°C by applying UV to NIR light with wavelength between 350 to 2000 nm in a hermetically sealed reactor.

In a preferred embodiment, the γ-ketoacid is acid to obtain γ-valerolactone.

In the present invention, a Ni-based catalyst refers to a catalyst comprising Ni (metallic) as an active metal.

In a preferred embodiment, the Ni-based catalyst is Raney Ni, which is highly stable and commercially available. The high stability of Raney Ni enabled several reaction cycles with the same catalyst without losing catalytic efficiency thanks to the mild reaction conditions. Moreover, the ferromagnetic properties of Raney Ni permit a straightforward catalyst recovery. The catalyst Raney Ni has a surface area BET between 80-100 $m^2g^{-1}$.

In another preferred embodiment, the Ni-based catalyst is a Ni-P based catalyst (comprises the elements Ni and P in a zero oxidation state), more preferably, a graphite/Ni-P catalyst, which comprise the elements Ni and P in a zero oxidation state deposited onto graphite, more preferably, Ni and P electrolessly deposited onto graphite flakes.

Electroless deposition (ELD) is the production of desired coatings from an aqueous solution without the application of the electrical external current or potential, allowing the uniform coating of a surface. In the process, metal ions are completely reduced and deposited over the surface under specific conditions from an appropriate reducing agent. The electroless solution itself is thermodynamically unstable but is stabilized by the various bath components. These components, apart from the metal salt and the reducing agent, are complexing agents, stabilizers, pH regulators and various other additives. Electroless deposition comprises two main steps:

I. Substrate sensitisation and activation. Generally, it involves the formation of Pd nuclei on the substrate surface, which act as activating agents for the electroless deposition.
II. Electroless deposition. The nickel (II) ions in the salt solution are reduced by an appropriate reducing agent, depositing a metal layer on the surface of the substrate, where Pd has acted as an activating agent. In this step, the Ni-P deposits at constant rate throughout the deposition time.

In a preferred embodiment, the graphite/Ni-P catalyst is obtained by electroless deposition method comprising:

a) A tin (II) salt, preferably $SnCl_2$, is solved in a solution of HCl, the solution is added to graphite flakes and it is treated with ultrasound for dispersion;
b) the graphite is then separated from the supernatant solution and added to solution of HCl containing a palladium (II) salt, preferably $PdCl_2$, and the resulting suspension is then subjected to ultrasonic treatment;
c) the palladium-decorated graphite is separated, an ammonia free Ni solution containing the reducing agent hypophosphite (electroless nickel plating solution - ammonia free- Nickelex) is added to the activated graphite and it is treated with ultrasound for the Ni-P deposition process.

In steps a) and b), the ultrasound treatment is preferably carried out at 35°C for 20 min. In step c), the ultrasound treatment is preferably carried out at a temperature between 70 and 90°C during 5 to 30 minutes, more preferably, for 10 min.

The graphite used in step a) can be previously treated with an acidic solution, for example, a solution of $H_2SO_4$

and /or a solution of $HNO_3$, preferably at 120-150°C, in order to hydrophilize the graphite surface, thus preparing the surface for subsequent activation and deposition processes

In a preferred embodiment, the graphite/Ni-P catalyst enhances the BET surface area of the graphite.

In a preferred embodiment, the graphite/Ni-P catalyst comprise graphite flakes (i.e.: graphite in the form of flakes), flakes having an average (mean number) lateral size going from 0.2 to 0.5 $\mu$m as determined by field emission scanning electron microscopy. The lateral size of the flakes is defined by the longest dimension of the flakes.

In the graphite/Ni-P catalyst, the composition of Ni-P preferably ranges from approximately 90 - 95% Ni (at.) and the corresponding 10% to 5% P (at.), as determined by mass spectrometry.

The graphite/Ni-P catalyst exhibits very high conversion rates at low reaction times and has demonstrated greater durability and stability throughout the reusability cycles, thus, being suitable catalyst for $\gamma$-valerolactone production. Said catalyst achieved nearly 100% conversion, with an optimum minimum reaction temperature of 100-120 °C.

In the present invention, the hydrogen source, also referred as H-source or hydrogenating agent, is isopropanol, which also acts as a solvent of the reaction. In particular, isopropanol has shown a significantly higher conversion efficiencies than other H-sources as, for example, formic acid. Moreover, it is increasingly recognized as a greener choice, due to the advances towards an industrial-scale production with a negative carbon footprint.

The procedure of the invention is carried out in a photothermal reactor that is composed of a hermetically sealed reactor or recipient enabling the light transmission. The reactor contains the solvent, reactants and Ni-based catalyst. The photothermal reactor has thermometers to control the temperature inside or at the surface of the reactor and a barometer to measure the pressure inside the reactor. As light source, any broadband or narrow band light source with wavelength between 350 to 2000nm can be employed, such as sunlight or high power lasers, preferably with emission in the 700 and 1100 nm range.

In a preferred embodiment, the concentration of the $\gamma$-ketoacidd in the solution of isopropanol is lower than 1.5 M, preferably is between 0.1 and 1.5 M.

In a preferred embodiment, the amount of catalyst is between 0.1 and 5%wt with respect to the $\gamma$-ketoacid.

In a preferred embodiment, the $\gamma$-ketoacid is contacted with a Ni-based catalyst and isopropanol under the reaction conditions for 60 to 120 min (reaction time).

The procedure has demonstrated nearly complete conversion (>95%) in a time equal or higher than 100 min, being this the preferred reaction time.

In a preferred embodiment, the temperature of the procedure of the invention is between 100 and 140°C, more preferably, between 120 and 140°C.

Two preferred embodiments are:

i) In a preferred embodiment, the procedure comprises contacting the $\gamma$-ketoacid, preferably levulinic acid, with the catalyst Raney Ni at a temperature between 130 and 150 °C, more preferably at 140°C during a time between 80 and 120 min.

ii) In a preferred embodiment, the procedure comprises contacting the $\gamma$-ketoacid, preferably levulinic acid, with the catalyst graphite/Ni-P at a temperature between 100 and 120 °C during a time between 80 and 120 min.

The described photothermal catalytic procedure presents notable advantages and improvements with respect to conventional or known method. Photothermal catalysis, which integrates light, heat and catalytic conversion, has enabled achieving excellent catalytic conditions even under moderate conditions, an improvement over thermocatalysis or photocatalysis processes.

Thus, the developed photothermal catalytic reaction enables a very high energy efficiency compared to conventional autoclave reactors, due to the direct heating of the Ni-based catalysts inside the reactor. The photothermal heating process exploits the highly damped plasmonic behavior of the Ni-based catalyst, characterized by broadband absorbance and high penetration of the electromagnetic fields of light inside the catalysis. This feature enables high light absorption (from UV to NIR) and reduced scattering with respect to typical plasmonic materials (e.g., Au, Ag), thereby providing very high photothermal efficiencies, even for large (micron-size) structures.

Therefore, the incident light is absorbed by the Ni-based catalysts inside the hermetically sealed reactor, generating intense local heating of the catalyst. As a result, the reactions can be achieved at lower global temperature of the reactants and solvents and, consequently, lower pressures. Moreover, as the heat is generated inside the reactor, the heat-losses can be minimized.

The procedure has demonstrated nearly complete conversion (>95%) for levulinic acid 0.5 M at a temperature of 140°C and a reaction time of 100 min in the photothermal reactor with Raney Ni as a catalyst, whereas in the conventional autoclave the reaction required 160°C and 240 min to reach 90% conversion efficiency.

When using graphite/Ni-P catalyst, conversions are practically 100% after 120 min of reaction at 120°C, whereas in the conventional autoclave the reaction at 180 °C takes 180 min to achieve the 100% conversion efficiency.

The reaction can be carried out several times with the same catalyst maintaining catalytic efficiency. As the Ni-based catalysis are ferromagnetic, the extraction of the catalyst is simplified by the magnetic trapping of the catalyst.

In conclusion, the photothermal procedure with Ni-based catalysts described in the present invention, which is considered a sustainable and economically and environmentally viable solution, enables:

- large reduction of the temperature and pressure necessary to drive the hydrogenation reaction,
- remarkable decrease of the reaction time,
- much lower energy consumption,
- safer reaction conditions,
- lower cost and higher abundance of the Ni-based catalysis, compared to other photothermal catalysts,
- broadband photothermal heating that enables using any wavelength from the UV to the infrared, being particularly suited for sunlight heating,
- easier extraction of the reaction products by magnetic trapping of the Ni-based catalyst.
- Use of the solvent as hydrogen source,

Unless otherwise defined, all technical and scientific terms used herein have the same meaning as commonly understood by one of ordinary skilled in the art to which this invention belongs. Methods and materials similar or equivalent to those described herein can be used in the practice of the present invention. Throughout the description and claims the word "comprise" and its variations are not intended to exclude other technical features, additives, components, or steps. Additional objects, advantages and features of the invention will become apparent to those skilled in the art upon examination of the description or may be learned by practice of the invention. The following examples, drawings are provided by way of illustration and are not intended to be limiting of the present invention.

## DESCRIPTION OF THE DRAWINGS

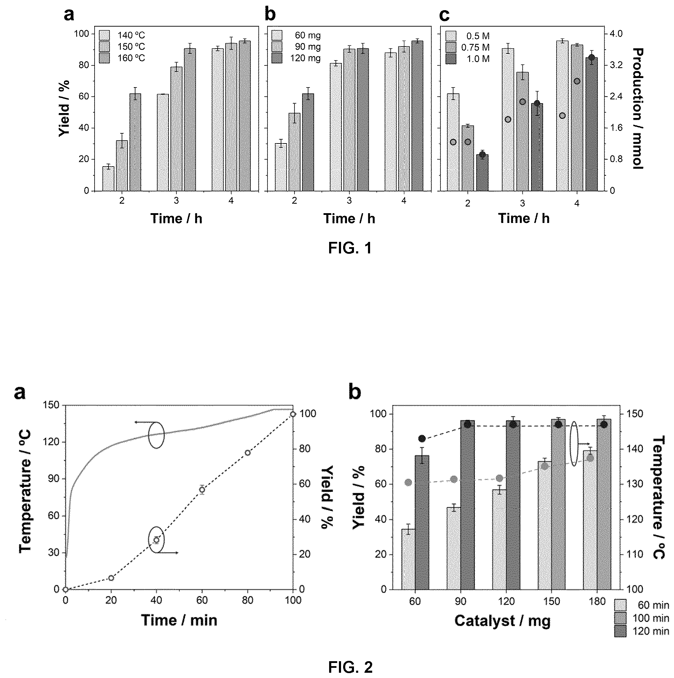

**Fig. 1.** Evaluation of the yield of $\gamma$-valerolactone provided under solvothermal conditions in conventional autoclave at different reaction times, depending on: (a) the temperature, (b) the amount of catalyst and (c) the initial concentration of levulinic acid (the total production in mmol is indicated with cycles and associated with the right axis). Unless otherwise stated, the reaction conditions imply: catalyst (Raney Ni) = 120 mg; $[LA]_0 = 0.5$ M; T = 160 °C. Each condition was performed in triplicate

**Fig. 2.** (a) Evolution of the temperature and yield of $\gamma$-valerolactone throughout a reaction time of 100 min using 120 mg of catalyst (Raney Ni); (b) yield of produced $\gamma$-valerolactone (bars) and maximum temperature (dots) depending on the amount of catalyst at three different times (60, 100 and 120 min). The reactions were conducted under laser-induced heating and solvothermal conditions in isopropanol, $[LA]_0 = 0.5$ M. The laser power was 18 W and it was fine-tuned when reaching 10 bars to ensure an inner pressure below it. Each result was performed in triplicate.

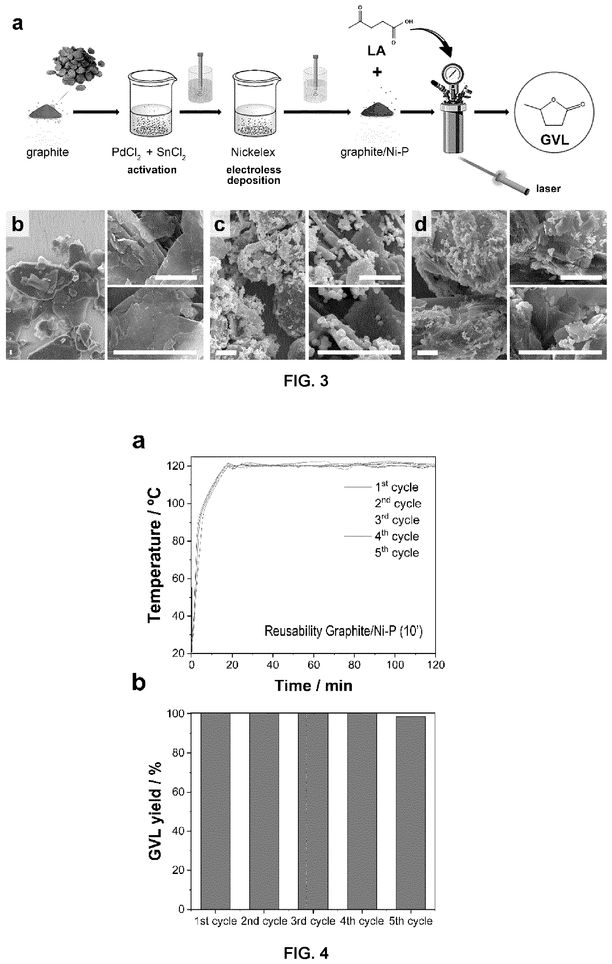

**Fig. 3.** (a) Synthesis scheme and FE-SEM micrographs of (b) graphite, (c) graphite/Ni-P 5' (d) graphite/Ni-P 10'. Scale bar: 100 nm.

**Fig. 4.** Temperature versus reaction time for (a) the reaction solution using 10 mg of graphite/Ni-P 10' catalyst, and (b) consecutive reusability cycles of 120 minutes for the GVL production at 120 °C using 10 mg of graphite/Ni-P 10' catalyst.

## Examples

In order to illustrate the invention, some examples carried out by the inventors using Raney Ni and graphite/Ni-P as catalyst are provided below.

## A) Examples using Raney Ni as catalyst

### 1.1. Synthesis and characterization

*1.1.1. Reagents and chemicals*

The reagents employed include levulinic acid (Thermo Scientific, 98%, CAS 123-76-2), formic acid (Sigma-Aldrich, >95%, CAS 64-18-6) and isopropanol (Panreac, technical grade, CAS 67-63-0). $\gamma$-Valerolactone (Thermo Scientific, 98%, CAS 108-29-2) was assessed for identification and calibration. As for the catalytic material, Raney$^®$ nickel microparticles (Sigma-Aldrich, 2800, slurry in 50% wt. $H_2O$, composition: 90% Ni, 9.5% Al, and 0.5% Fe, CAS 7440-02-0) with an expected specific surface area ranging between 80-100 $m_2$ $g^{-1}$ have been selected. All reagents were used as received.

### 1.1.2. Instrumentation

The techniques utilized for the characterization of the catalytic material are herein specified. The crystalline structure of the as-received and the reutilized Raney$^®$ Ni microparticles was elucidated via X-ray diffraction (XRD). Regarding their phase constitution and morphology, were analyzed through field-emission scanning electron microscopy (coupled with energy-dispersive X-ray spectroscopy (EDX).

### 1.1.3. Synthetic procedure of GVL

The initial synthetic experiments were conducted under hydrothermal conditions (for comparative purposes) in a Teflon reactor placed into a 50 mL autoclave, utilizing 4 mL of the reaction solution (0.5 M of levulinic acid and isopropanol) alongside the appropriate amount of catalyst. Even though the catalyst was weighted as a slurry for each experiment, the quantities specified throughout this work refer to the weight of the dry microparticles, estimated as a 1:1 microparticle:$H_2O$ wt./wt. ratio. The second stage involved solvothermal conditions and were similarly carried out with 4 mL of the reaction solution (0.5 M of levulinic acid in isopropanol). Prior to sealing the reactor, all solutions underwent a 5-minute purge with nitrogen. Subsequently, the autoclave was introduced into the pre-stabilized stove set at the designated temperature. Upon completion of the programmed reaction time, the reactor was removed from the stove, allowed to cool for 20 minutes, and finally submerged into water to speed the cooling process to room temperature. The resulting reaction mixture was diluted with Milli-Q water and separated from the catalyst with the assistance of an external magnet.

For synthetic experiments conducted under light (laser) irradiation (procedure of the present invention), the appropriate amount of catalyst and 4 mL of the reaction solution (0.5 M of levulinic acid in isopropanol) were introduced into a glass tiny clave reactor of 25 mL equipped with a manometer and purged for 5 min with nitrogen. The temperature of the reactor was monitored throughout the experiment with an infrared thermometer, which enabled the control of the reactor temperature by tuning the laser power. The light-mediated heating process was controlled by tuning the output power of a fiber coupled laser diode with emission at 915 nm, which was collimated in a spot of 2 cm diameter. The laser power was modulated to reach and keep specified T set point and to ensure a pressure below 10 bars inside the autoclave reactor. The laser power was initially set at 18 W until reaching the desired T value, which was automatically maintained by fine-tuning the laser power. For the reusability tests, the catalyst was washed with isopropanol thrice and dried with nitrogen. The tinyclave reactor was immediately filled with 4 mL of the reaction solution, purged with nitrogen for 5 min and sealed.

The composition of each sample was analyzed by means of liquid chromatography (HPLC) coupled with a PDA detector and a mass detector. Prior to injection into the chromatograph (1.5 $\mu$L), the solution was centrifugated at 6000 rpm for 15 minutes and was filtered using 0.22 $\mu$m syringe filters to eliminate any solid residue. The mobile phase comprised a mixture of water and acetonitrile (starting ratio of 9:1 v/v) with 0.1% formic acid, and an ACQUITY CSH C18 column (1.7 $\mu$m, 3.0×50 mm) was selected. The concentration of the reagent and products were quantified after calibration of the equipment, which enabled the calculation of the conversion of levulinic acid (LA) and the yield of $\gamma$-valerolactone (GVL) using the following equations:

$$Conversion = ([LA]_0 - [LA]_t) \times 100 \, / \, [LA]_0$$

$$Yield = [GVL]_t \times 100 \, / \, [LA]_0$$

in which $[LA]_0$ and $[LA]_t$ denote the initial and final concentrations of levulinic acid, respectively, and $[GVL]_t$ represents the final concentration of $\gamma$-valerolactone. These calculations permitted the assessment of the efficiency and yield of the synthesis process.

## 1.2. Comparison between H-sources

As a preliminary step, the synthetic conditions with two hydrogenating agents, i.e. formic acid and isopropanol, employing Raney nickel as catalyst under hydrothermal conditions were optimized. The comparison between formic acid and isopropanol was based on evaluating the yield of $\gamma$-valerolactone considering two key experimental parameters: temperature and reaction time. The initial concentration of levulinic acid was set at 0.5 M, while maintaining an excess of 4 equivalents of either formic acid or isopropanol. The yield of γ-valerolactone over time at different temperatures indicates that the utilization of formic acid requires a higher temperature to proceed than isopropanol, with an optimal performance placed between 200-220 °C. At 220 °C, significant progress is observed after 8 hours of reaction, culminating in a maximum conversion and yield of 93% and 75%, respectively. When the reaction is conducted at 180 °C, however, the yield drops below 10% even after 24 hours. Isopropanol as the H-source offers a remarkable reduction in reaction time. Notably, the reaction proceeds in just 2 hours at 220 °C, yielding a 93% conversion and 81% yield. This implies a reduction of 6 hours in the reaction time, with even a 6% increase in yield with respect to the optimal conditions achieved with formic acid at the same temperature.

Isopropanol permits a substantial decrease in the operating temperature. The reaction attains yields nearing 80% after 5 hours at 160°C, underscoring the appropriateness of isopropanol in this reaction. Overall, isopropanol as a hydrogen source promotes a faster and more sustainable procedure, arising as the optimal choice for this synthetic strategy. Furthermore, the lower calorific value of isopropanol presents an additional advantage, particularly in the light-mediated heating system we propose. Consequently, it has been tested in a higher excess, capitalizing on its potential as a solvent.

## 1.3. Solvothermal conditions (for comparative purposes)

Taking into account the assets observed for isopropanol, its use as solvent, suggests promising conversion rates under milder conditions. The production of γ-valerolactone under solvothermal conditions varying the aforementioned factors, namely temperature, amount of catalyst and initial concentration of levulinic acid were studied. The standard conditions were set at 160 °C, 120 mg of Raney Ni and an initial concentration of levulinic acid of 0.5 M (Fig. 1).

As observed, the results derived from using isopropanol as solvent outpace those obtained with a molar proportion 1:4 (LA:IPA (isopropanol)) ratio at 160 °C, escalating from 60 to 96% yield in 4 h. Temperature remains a determining factor from a kinetic point of view, proceeding considerably faster at 160 °C than at lower temperatures (Figure 1a). This is especially noticeable when carried out for 2 h, resulting in yields of 15 and 62% at 140 and 160 °C, respectively. It should be mentioned, though, that the reaction is perfectly accomplishable at 140 °C in 4 h, with a yield of 91%. Therefore, employing isopropanol as solvent also enables an additional reduction of 20 °C, considering that the molar ratio 1:4 (LA:IPA) ratio only provides a 60% in the same period. Secondly, the dependence of the yield upon the ratio of catalyst was equally analyzed (Figure 1b). As expected, economizing the amount of catalyst slows down the process, but the yield provided after 4 h is almost unaffected. In fact, the step of scaling it down from 120 to 90 mg provides analogous results at 3 and 4 h, with a yield of γ-valerolactone surpassing 90%. Lastly, Figure 1c represents the yield and production in mmol of γ-valerolactone with respect to different concentrations of reagent. The production data provides a closer insight to the capacity of the catalyst to support the reaction. As observed, it affords analogous values after 2 h regardless of the concentration, suggesting that the production is limited to 1.2 mmol with 120 mg of catalyst. The same applies to 3 h, with a maximum production of ca. 2.3 mmol of γ-valerolactone. A concentration of 1 M of LA escalates the production to 3.4 mmol after 4 h, with a respectable yield of 85%. Hence, the augment in levulinic acid does not restrict the development of the reaction nor accelerates it.

## 1.4. Photo-thermo-catalytic conditions (conditions of the present invention)

The photo-thermo-catalytic conversion from levulinic acid to γ-valerolactone was carried out under infrared-light irradiation with a fiber-coupled laser diode (λ = 915 nm) into a glass tinyclave reactor. The temperature values were recorded with an integrated infrared thermometer. In the photothermal reactor, the heating rate could depend on two main factors: the laser power and the amount of catalyst. The optimal laser power range was established between 15 W and 18 W, as lower values were insufficient to reach the required temperature range in a practical extent of time. Regarding the amount of catalyst, the corresponding heating curves proceed at a similar pace on the early stage of the process. Nevertheless, the system requires a certain amount of catalyst to maintain a steady increase of the temperature while the reaction is taking place. Specifically, with the addition of 120 mg of catalyst, pure isopropanol takes ca. 20 min to attain 140°C, whilst a solution containing 0.5 M of levulinic acid needs 80 min. The yield of γ-valerolactone was determined at different reaction times throughout a 120-minutes period (Figure 2a) at a fixed current intensity. The heating curve is also represented to correlate the progress of the reaction with the temperature.

The evolution of the temperature during this stage is highlighted on the corresponding graph. As observed, the reaction advances at a prompter pace when light-assisted than in a conventional heating. Remarkably, it only requires 60 min to furnish a 57% conversion to γ-valerolactone, with a maximum temperature of 130 °C, and it required only 100 min upon completion, with a maximum temperature of 145 °C. These initial results also acknowledge that the reaction admits lower working temperatures to take place employing this strategy. The impact of the amount of catalyst on both the yield and maximum temperature was also surveyed from 60 mg to 180 mg using the same illumination conditions (Figure 2b). The reaction was conducted for 60 and 120 min (100 min in the cases of 150 and 180 mg because of their faster completion). The yield obtained after 1 h perfectly correlates with the amount of catalyst, spanning from 35 to 79%. The difference between maximum temperatures at that point is not compelling, suggesting that the yield variation is principally related to the available catalyst. Except for the reaction involving the least quantity of catalyst (i.e. 60 mg), all reactions were accomplished within 2 h, with yields beyond 96% reaching similar maximum temperatures. In fact, 120 mg or higher permit almost quantitative conversions in a period of 100 min. Hence, such heating strategy grants a more optimized process by focalizing the heat onto the surface of the catalytic material. With these in mind, the next step involved working at a settled temperature. Based on the progression of the reaction with respect to the temperature, we selected 130°C as the optimal temperature for a 2-hour process. For this, the current intensity was modulated manually regulated to maintain the maximum value once the system reaches it, which occurred after ca. 1 h. Hence, the energy consumption invested on the heating diminishes substantially during this second stage. When carried out at 130 °C, even if the system falls short in providing $\gamma$-valerolactone quantitatively, it yields it in an excellent 93%. Rising to 132 °C translates into an even higher value of 97%. Under these conditions, the reusability of the catalyst was also tested as a key aspect to ensure the sustainability of the process, since the catalytic material assists both the reaction and the heating of the system. The results withstand the high potential of this synthetic strategy, exhibiting a remarkable reusability that grants a yield exceeding 90% and low fluctuation after consecutive cycles. Besides, the magnetic character of the catalyst permits an easy recover and separation. The stability of the catalyst was also investigated by quantifying the metal ions leaked into the reaction medium via ICP-MS, being the detected concentrations of nickel very low.

The energy consumption of the system, operated at a fixed temperature, was estimated on the basis of the current intensity applied throughout the experiment. The results developed so far pave the way to the implementation of a light-driven thermo-catalytic strategy for a greener and scalable production of $\gamma$-valerolactone, employing Raney Ni as a commercial non-precious platform.

**B) Examples using graphite/Ni-P as catalyst**

The catalysts were functionalized via a Ni-P electroless deposition process. For the effective catalysts, different coverages thicknesses were tested.

**1.1. Electrosynthesis of Ni-P-based catalyst**

Graphite flakes (Sigma-Aldrich CAS 7782-42-5) were used as substrates for Ni-P electroless deposition to prepare the graphite/Ni-P catalyst, 0.375 g of $SnCl_2$ (15 g/L solution) was gradually dissolved in 25 mL of 2 M HCl at 35 °C due to the low solubility of the compound. Once the sensitization solution was prepared, it was added to 50 mg of graphite and treated with ultrasound for 20 minutes under the conditions specified in Table 1. At the end of the process, the graphite was separated from the supernatant solution by centrifugation and then added to a 25 mL solution of 0.2 g L$^{-1}$ PdCl$_2$ in 2 M HCl at 35 °C. The resulting suspension was then subjected to ultrasonic treatment for 20 min, and the palladium-decorated graphite was separated from the supernatant by centrifugation. Next, a 90°C Nickelex solution (electroless nickel plating solution - ammonia free; Sigma-Aldrich, product number: 901655) was added to the activated graphite and treated with ultrasound for the Ni-P deposition process. In this experiment the following times were tested: 5 min (identified as graphite/Ni-P (5')) and 10 min (identified as graphite/Ni-P (10')). Finally, to remove inorganic and organic residues, the functionalized graphite was filtered and washed with Milli-Q water and ethanol. The final product was then dried.

For the graphite substrate, a previous acidic treatment was also attempted. This involved adding 27 mL of $H_2SO_4$ 96% and 18 mL of $HNO_3$ 65% to 0.25 g of graphite in an autoclave. The mixture was then treated for six hours at 140 °C in the oven. This process was carried out in order to hydrophilize the graphite surface and enable the electroless deposition process to be performed more efficiently. Figure 3a illustrates the global procedure.

It is important to note that the PdCl$_2$ solution could be reused up to three times during the catalyst preparation process, which enhances the cost-effectiveness of the procedure and reduces the quantity of reactants required. Also, solution reusability is possible with Nickelex solution certain number of times, depending on the surface and deposition times.

*Table 1.* *Ultrasonic Homogenizer conditions for the different steps of the catalysts tested*

| Solution | Substrate | T-Run [min] | Power [%] | T-On [s] | T-Off [s] | Temp [°C] |
|---|---|---|---|---|---|---|
| SnCl$_2$ | Graphite | 20 | 35 | 2.0 | 1.0 | 35 |
| PdCl$_2$ | Graphite | 20 | 35 | 2.0 | 1.0 | 35 |
| Nickelex | Graphite | 5 | 35 | 2.0 | 1.0 | 70 and 90 |
| Nickelex | Graphite | 10 | 35 | 2.0 | 1.0 | 70 and 90 |

## 1.2. Characterization of Ni-P-based catalysts

The surface morphology and architecture of catalysts were analyzed by using a field emission scanning electron microscopy equipped with an energy dispersive X-ray (EDX) detector. Figure 3b illustrates that untreated graphite flakes exhibited a layered structure with smooth surfaces, indicative of their inherent crystalline nature. The layers were clearly delineated, exhibiting typical flake-like characteristics with sharp edges and flat planes, which were often intermingled with minor surface irregularities. With regard to size, despite the existence of a heterogeneous distribution, the flakes were predominantly between 0.2 and 0.5 $\mu$m. The changes in surface morphology demonstrates that the coating was successfully applied using the ELD process (Figures 3c, 3d). For graphite flakes, the coating by the electroless bath after 5 min was partial (Figure 3c). However, longer deposition time resulted in a higher coating for the substrates in Figure 3d, which underwent 10 minutes of Nickelex synthesis. It can be observed that the substrates that have been covered with Ni-P have a larger surface area compared with the non-recovered ones. This is due to the addition of the Ni-P deposit, which is composed of small nanoparticles, thus considerably increasing the surface area. After 5 minutes of electroless deposition, the graphite flakes were partially covered with granular structures. After 10 minutes of electroless deposition, the Ni-P coating formed a continuous layer, although some areas exhibited a higher density of granular deposits. Irrespective of the deposition time, the composition of Ni-P deposits typically ranged from approximately 89% to 95% Ni (at.) being P the codeposited element. In order to study the distribution of the different elements of the catalyst's surface, element mapping of the coated catalysts was performed. Nickel was deposited on a larger surface than phosphorus. It can be concluded that oxygen was not present on the substrate, which indicates that neither the graphite nor deposited Ni-P film were oxidized during the process .

A thermogravimetric analysis (TGA) was conducted covering a temperature range from room temperature to 200 °C at a heating rate of 5 °C/min in air atmosphere to examine the percentage of mass lost as a function of temperature. The thermograms displaying the percentage of decomposition of the graphite and both prepared catalysts graphite/Ni-P 5' 10' showed that both 5' and 10' substantially remained unchanged in mass, with negligible losses of 0.03% and 0.01% respectively. In contrast, the graphite experienced a slight oxidation of 0.01 %. Generally, all samples can be considered thermally stable within the temperature range utilized in this study. The materials were characterized by X-ray diffraction (XRD). XRD analysis revealed that the Ni-P electroless deposition process resulted in a mixture of amorphous and crystalline phases, with the most relevant being nickel phosphides. The elevated temperatures generated at the substrate interface during the ultrasonic electroless NiP deposition process facilitated the deposition of Ni-P in crystalline form. The presence of peaks corresponding to the graphite substrate (reference code: 00-008-0415) verifies that the hexagonal graphite structure remains intact after the Ni-P electrodeposition process. The structure of Ni-P deposits was typically amorphous or nanocrystalline, depending on the phosphorus content and deposition conditions. In general, a broad hump was observed around $2\theta = 40°\text{-}50°$, which indicates the presence of an amorphous or poorly crystalline Ni-P phase. This is a typical characteristic of Ni-P coatings prepared by electroless deposition, where a higher phosphorus content leads to a greater degree of amorphous characteristics. In the case of graphite/Ni-P 5', the XRD pattern exhibited the characteristic peaks of graphite, in addition to peaks corresponding to cubic metallic Ni (reference code: 00-004-0850), approximately at $2\theta \approx 44.5°$ (111), 51.8° (200), and 76.4° (220). Nickel phosphides such as the tetragonal $Ni_3P$ (reference code (JCPDS): 00-034-0501) as well as the hexagonal $Ni_5P_2$ (reference code (JCPDS): 00-017-0225) crystalline phases were also identified. In the case of graphite/Ni-P 10' were also identified small peaks that were consistent with $Ni_{12}P_5$ (reference code (JCPDS): 00-022-1190). It should be noted that the differentiation between these phases was challenging due to the proximity of their peaks, which frequently overlap. Importantly, no peaks characteristics of graphite oxide were detected. This analysis provided confirmation of the electroless deposition of Ni-P.

The total amount of Ni-P electrolessly deposited onto the graphite substrate was determined using inductively coupled plasma optical emission spectroscopy (ICP-) for ppm scale quantification and inductively coupled plasma mass spectrometry (ICP-MS) for ppb scale quantification. Prior to analysis, the Ni-P deposited was dissolved using concentrated nitric acid. Additionally, the stability of graphite/Ni-P catalysts during the photo-thermocatalytic production of GVL was assessed by measuring the Ni and P concentrations released in the liquid phase during the reaction.

The surface area estimated of the different catalysts was highly dependent on the NiP deposited. The BET surfaces areas obtained were 75.3 m$^2$/g for the graphite substrate, 101.2 m$^2$/g for the graphite/Ni-P (5') and 92.2 m$^2$/g for the graphite/Ni-P (10'). It can be observed that the electroless process had significantly increased the surface area of the C substrate. The reduction in the surface area of the graphite/NiP (10') catalyst in comparison to the graphite/Ni-P (5') catalyst could be due to the agglomeration of Ni-P particles during the electroless deposition process.

These characterizations serve to validate the electroless deposition of Ni-P on graphite and provide insights into its chemical composition and structural properties.

## Green photo-thermocatalytic production of GVL

Both graphite catalysts underwent testing under identical parameters, operating at 120 °C and varying reaction times. These experiments utilized a laser as the energy source for the reaction, which heats the catalyst, causing heat transfer from the catalyst to the solution. The photo-thermocatalytic conversion of levulinic acid to GVL was checked under near infrared laser irradiation ($\lambda$ = 915 nm) within a glass tinyclave reactor. Simultaneous temperature monitoring was conducted using an integrated probe. Within this system, the heating was determined as a function of two primary factors: the intensity of the current applied to the laser and the quantity of catalyst employed. The use of light as energy source to study the production of GVL is a key step for the industrial scale-up of the process, allowing the potential reduction of emissions and process costs.

These experiments were performed in order to determine the production and efficiency of each catalyst in a photothermal reaction. On the other side, the optimal conditions for these experiments were determined taking into account the autoclave tests results.

The overall experimental system comprises a laser, a reactor, a laptop to control light power, a current source and a thermometer probe. The reactor was a glass tinyclave reactor protected with a thermal isolation film that maintains the temperature, as well as a lid equipped with a gauge and a barometer with a valve that is opened at 10 bars as a safety measure. The laser was positioned under the reactor, with a series of mirrors directing the light toward the reactor with a spot diameter of 2 cm. All the parameters were controlled from a computer application (AeroDIODE Control Software). During the reaction, the temperature was monitored continuously.

The temperature, reaction volume and reagent concentration were kept constant. The reaction temperature was established at 120 °C. Different reaction times were tested: 40 min, 80 min and 120 min. The experiment and conditions employed are presented in Table 3. There was no need to apply $N_2$ gas flow since it was posteriori established that the residual air did not affect the structure or surface of the catalyst.

*Table 3. Conditions studied for GVL production using the laser as energy source*

| Catalyst name | Time [min] | Temperature [°C] | Amount of catalyst [mg] | LA 0.5 M [mL] |
|---|---|---|---|---|
| NiP 5' | 40 | 120 | 10 | 4 |
| NiP 5' | 80 | 120 | 10 | 4 |
| NiP 5' | 120 | 120 | 10 | 4 |
| NiP 10' | 40 | 120 | 10 | 4 |
| NiP 10' | 80 | 120 | 10 | 4 |
| NiP 10' | 120 | 120 | 10 | 4 |

In order to conduct the experiments, the reagent (LA 0.5 M in IPA) and the catalyst were added to the reactor. Throughout the course of each experiment, the laser intensity was modulated to maintain the temperature of the reactor constant. The initial intensity was 16.5 W until reaching the desired temperature and then the power was controlled by the software to maintain the fixed working temperature. The pressure of the inside the reactor did not exceeded 5 bar. After heating for the determined times, the reactor was cooled to room temperature and the solution was diluted with 5 mL of Milli-Q water for the posterior HPLC analysis.

The composition of each sample was analyzed using liquid chromatography (HPLC) coupled with a PDA detector and a mass detector. Before injection into the chromatograph (1.5 $\mu$L), the solution underwent centrifugation at 6000 rpm for 15 minutes and filtration using 0.22 $\mu$m syringe filters to remove any solid residue. The mobile phase consisted of a mixture of water and acetonitrile (initial ratio of 9:1 v/v) with 0.1% formic acid, and an ACQUITY CSH C18 column (1.7 $\mu$m, 3.0$\times$50 mm) was employed.

The concentration of reagents and products was quantified after equipment calibration, allowing for the calculation of levulinic acid conversion and $\gamma$-valerolactone yield using the following equations:

$$Conversion = \frac{[LA]_0 - [LA]_t}{[LA]_0} x100$$

$$Yield = \frac{[GVL]_t}{[LA]_0} x100$$

where $[LA]_0$ and $[LA]_t$ represent the initial and final concentrations of levulinic acid, respectively, and $[GVL]_t$ denotes the final concentration of $\gamma$-valerolactone. These calculations enabled the evaluation of synthesis process yield.

Figure 4a shows the evolution of the temperature of the system during 120 minutes for the reaction solution.

Regarding the GVL production, the conversion efficiency progressively increased to 100% with the reaction time for the graphite/Ni-P (10') catalyst, while the graphite/Ni-P (5') catalyst reached a yield of 98.6% at the same point. At all reaction times, the yields of the graphite/Ni-P (10') catalyst were consistently higher than the corresponding of the graphite/Ni-P (5') catalyst. These results can be attributed to the greater coverage in the graphite/Ni-P (10') catalyst.

The reusability of the two tested graphite/Ni-P catalysts (5' and 10') was investigated to evaluate the efficiency, performance, and stability of the catalysts after different cycles and to determine the possible loss of the activity. Additionally, the release of nickel and phosphorous from the catalyst was evaluated.

The graphite/Ni-P 5' and graphite/Ni-P 10' catalysts were subjected to a total of five reaction cycles of 120 minutes each. After each cycle, the catalyst was separated from the supernatant by centrifugation and washed three times with IPA to remove any possible traces of GVL that might affect the subsequent results. The catalysts prepared after 10' of electroless (Figure 4b) demonstrate that the reaction yield remained constant over the cycles (Figure 4b), unlike the 5' coated catalysts. This difference may be attributed to the lower stability of the Ni-P coating in the 5' catalyst, which could led to a loss of activity due to poisoning or aging. The surface chemical composition of the catalysts, both before and after the reaction, was characterized to demonstrate the surface integrity and to assess any alterations in the elemental composition of the surface. It can be observed that for both catalysts, the Ni-P coating deposited by electroless deposition was maintained after cycling. No appreciable morphological differences were observed between the different catalysts before and after use. The catalysts were also analyzed by XRD, and no appreciable changes were observed with respect to the initial catalyst.

The nickel and phosphorus release to the solution was characterized by ICP-MS analysis, being the amount released from the 5' catalyst significantly higher than that of the 10' catalyst. However, it was always lower than 0.06% of the total mass of nickel and/or phosphorus.

These findings indicate that the catalyst did not experience significant structural or compositional alterations during the reaction demonstrating its stability and durability.

## Claims

1. Procedure for the conversion of a γ-ketoacid to the corresponding γ-lactone via photothermal catalysis comprising contacting the γ-ketoacid with a Ni-based catalyst and with isopropanol, which is used as a hydrogen source and solvent, and photothermally heating at a temperature between 100 and 150°C by applying UV to NIR light with wavelength between 350 to 2000 nm in a hermetically sealed reactor.

2. Procedure, according to claim 1, wherein the Ni-based catalyst is Raney Ni.

3. Procedure, according to claim 1, wherein the Ni-based catalyst is a Ni-P-based catalyst.

4. Procedure, according to claim 3, wherein the Ni-based catalyst is a graphite/Ni-P catalyst.

5. Procedure, according to any of the preceding claims, wherein sunlight radiation is used as light source.

6. Procedure, according to claims 1 to 3, wherein a laser with emission in the range between 450 nm to 1300 nm is used as light source.

7. Procedure, according to any of the preceding claims, wherein the concentration of the γ-ketoacid in the solution of isopropanol is between 0.1 and 1.5 M.

8. Procedure, according to any of the preceding claims, wherein the amount of catalyst is between 0.1 and 5 %wt with respect to the γ-ketoacid.

9. Procedure, according to any of the preceding claims, wherein the reaction time is between 60 and 120 min.

10. The procedure, according to any of the preceding claims, wherein the temperature of the procedure is between 100 and 140°C.

11. Procedure, according to claim 1, wherein the procedure comprises contacting the γ-ketoacid with the catalyst Raney Ni photothermally heated at a temperature between 130 and 150 °C, more preferably at 140°C, during a time between 80 and 120 min.

12. Procedure, according to claim 1, wherein the procedure comprises contacting the $\gamma$-ketoacid with the catalyst graphite/Ni-P photothermally heated at a temperature between 100 and 120 °C during a time between 80 and 120 min.

13. The procedure, according to any of the preceding claims, wherein the $\gamma$-ketoacid is levulinic acid and the obtained $\gamma$-lactone is $\gamma$-valerolactone.

FIG. 1

FIG. 2

FIG. 3

FIG. 4

Europäisches
Patentamt

European
Patent Office

Office européen
des brevets

**EUROPEAN SEARCH REPORT**

Application Number

EP 24 38 3185

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | YU ZHIQUAN ET AL:  "Catalytic Transfer Hydrogenation of Levulinic Acid to [gamma]-Valerolactone over Ni 3 P-CePO 4 Catalysts", INDUSTRIAL & ENGINEERING CHEMISTRY RESEARCH, vol. 59, no. 16, 18 March 2020 (2020-03-18), pages 7416-7425, XP093261719, ISSN: 0888-5885, DOI: 10.1021/acs.iecr.0c00257 * Schemes 1 and 2, chapter 4. Conclusions; figures 5,8 * | 1-13 | INV. C07D307/33 C07C67/08 |
| A | YUN WAN-CHU ET AL:  "Microwave Irradiation-Enhanced Catalytic Transfer Hydrogenation of Levulinic Acid to [gamma]-Valerolactone Using Ruthenium: A Comparative Study with Conventional Heating Processes", WASTE AND BIOMASS VALORIZATION, SPRINGER NETHERLANDS, NL, vol. 11, no. 6, 25 February 2019 (2019-02-25), pages 2783-2793, XP037114306, ISSN: 1877-2641, DOI: 10.1007/S12649-019-00623-Y [retrieved on 2019-02-25] * chapter Conclusions; figures 1,2,4-6 * | 1-13 | |
| | -/-- | | TECHNICAL FIELDS SEARCHED (IPC) C07D C07C |

The present search report has been drawn up for all claims

| Place of search | Date of completion of the search | Examiner |
|---|---|---|
| Munich | 21 March 2025 | Guspanová, Jana |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches
Patentamt

European
Patent Office

Office européen
des brevets

## EUROPEAN SEARCH REPORT

**Application Number**

EP 24 38 3185

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | MENG YE ET AL: "Surface-active site engineering: Synergy of photo- and supermolecular catalysis in hydrogen transfer enables biomass upgrading and H2 evolution", CHEMICAL ENGENEERING JOURNAL, ELSEVIER, AMSTERDAM, NL, vol. 452, 29 September 2022 (2022-09-29), XP087216412, ISSN: 1385-8947, DOI: 10.1016/J.CEJ.2022.139477 [retrieved on 2022-09-29] * Table 1, entries 3, 11-14 * ----- | 1-13 | |

TECHNICAL FIELDS
SEARCHED (IPC)

The present search report has been drawn up for all claims

| Place of search | Date of completion of the search | Examiner |
|---|---|---|
| Munich | 21 March 2025 | Guspanová, Jana |

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **VALENTINI, F.** ; **MARROCCHI, A.** ; **VACCARO, L.** *Adv. Energy Mater.*, 2022, vol. 12, 2103362
- **HIJAZI, A.** ; **KHALAF, N.** ; **KWAPINSKI, W.** ; **LEAHY, J. J.** *RSC Adv.*, 2022, vol. 12, 13673-13694
- **FANG, S.** ; **HU, Y. H.** *Chem. Soc. Rev.*, 2022, vol. 51, 3609-3647
- **ZHANG, J.** ; **CHEN, H.** ; **DUAN, X.** ; **SUN, H.** ; **WANG, S.** *Mater. Today*, 2023, vol. 68, 234-253

- *CHEMICAL ABSTRACTS*, 123-76-2
- *CHEMICAL ABSTRACTS*, 64-18-6
- *CHEMICAL ABSTRACTS*, 67-63-0
- *CHEMICAL ABSTRACTS*, 108-29-2
- *CHEMICAL ABSTRACTS*, 7440-02-0
- *CHEMICAL ABSTRACTS*, 7782-42-5